(19) Europäisches Patentamt
European Patent Office
Office européen des brevets

(11) Publication number: 0 347 123 A2

(12) EUROPEAN PATENT APPLICATION

(21) Application number: 89305858.6

(22) Date of filing: 09.06.89

(51) Int. Cl.[4]: C07D 211/70 , C07D 211/14 , C07D 405/04 , C07D 211/22 , C07D 313/12 , A61K 31/335 , A61K 31/445

The title of the invention has been amended (Guidelines for Examination in the EPO, A-III, 7.3).

Claims for the following Contracting States: ES + GR

(30) Priority: 17.06.88 US 207839
17.06.88 US 207840

(43) Date of publication of application: 20.12.89 Bulletin 89/51

(84) Designated Contracting States:
AT BE CH DE ES FR GB GR IT LI LU NL SE

(71) Applicant: FISONS CORPORATION
Jefferson Road P.O. Box 1710
Rochester, New York 14603(US)

(72) Inventor: Griffith, Ronald C.
41 Northfield Gate
Pittsford, NY 14534(US)
Inventor: Napier, James J.
203 Lake Shore Drive
Lindenhurst Illinois 60046(US)

(74) Representative: Wright, Robert Gordon McRae
FISONS plc 12 Derby Road
Loughborough Leicestershire LE11 0BB(GB)

# (54) Dibenzo-cycloheptenyl, -cycloheptyl and -oxepinyl amines having antihistaminic properties.

(57) There are provided compounds of formula I:

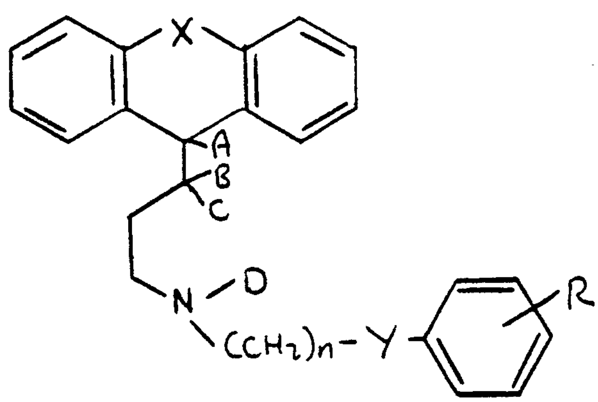

I

wherein:
X represents $-CH_2O-$, $-CH_2CH_2-$ or $-CH=CH-$;
either A represents -OH and B represents hydrogen, or A and B taken together form a second bond between the carbons to which they are attached;
either C represents hydrogen and D represents C1 to 6 alkyl, or C and D form a saturated two carbon chain.

EP 0 347 123 A2

Y represents $-CH_2-$, $-C(=O)-$, $-CH(OH)-$, -S-, -NH-, -0-, or $-NHCH_2CH_2-$;
R represents C1 to 6 alkyl, $-C(CH_3)_2CH_2OH$, $-C(CH_3)_2CO_2H$, $-C(CH_3)_2COOR'$; and may be at the 2, 3 or 4 position of the benzene ring relative to the rest of the molecule,
R′ represents C1 to 6 alkyl
and n represents an integer between 3 and 6.

**Novel Dibenzo-cycloheptenyl,-cycloheptyl and -oxepinyl amines having antihistaminic properties.**

## Background of the Invention

This invention relates novel pharmaceutical compounds, processes for their preparation and compositions containing them.

The utility of antihistamine compounds (histamine $H_1$ antagonists) as a treatment for the alleviation of the symptoms of allergic disorders has been long recognized. However, due to their effects on the central nervous system, numerous side effects, most notably sedation, are observed with these agents (Douglas, W.W., in "The Pharmacological Basis of Therapeutics", 6th ed., Gilman, A.G. et al, Ed., Macmillan: New York, 1980, pp 622-632), for example, cyproheptadine (4-(5H-dibenzo[a,d]cyclohepten-5-ylidene)-1-methylpiperidine), and the tricyclic antidepressant doxepin (3-dibenz[b,e]oxepin-11(6H)-ylidene-N,N-dimethyl-1-propanamine), which is a potent antagonist of histamine $H_1$ receptors but is known to cause sedation (Figge, J.; Leonard, P. and Richelson, E: Eur J. Pharm., 1979, 58, 479-483). Because of its activity in the central nervous system, the use of doxepin in the treatment of allergic disorders has been limited to topical treatment (Bernstein, J.E and Endicott, C.J.: Eur. Pat. Appl. EP93373 and Bernstein, J.E. US Pat No. 4395420.

## Brief Summary of the Invention

This invention provides compounds of formula (I):

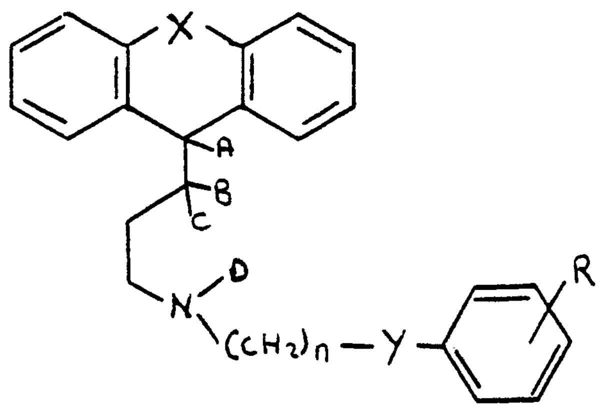

I

wherein X represents $-CH_2O-$, $-CH_2CH_2-$ or $-CH=CH-$;
either A represents -OH and B represents hydrogen, or A and B taken together form a second bond between the carbons to which they are attached;
either C represents hydrogen and D represents C1 to 6 alkyl, or C and D form a $CH_2CH_2$ chain.
Y represents $-CH_2-$, $-C(=O)-$, $-CH(OH)-$, -S-, -NH-, -0-, or $-NHCH_2CH_2-$;
R represents C1 to 6 alkyl, $-C(CH_3)_2CH_2OH$, $-C(CH_3)_2COOR'$, in which $R'$ represents hydrogen or C1 to 6 alkyl
and n represents an integer between 3 and 6.

This invention also relates to pharmaceutically acceptable acid addition salts of the compounds of formula I.

## Detailed Description of the Invention

It has now been discovered that the compounds of this invention possess antihistaminic activity with a low potential for sedation.

Some of the compounds of formula I above are capable of existing in enantiomeric and diastereoisomeric forms. This invention relates to all enantiomeric and diastereomeric forms of compounds of formula I as well as mixtures thereof. Additionally, some of the compounds of formula I are capable of existing as cis and trans olefinic isomers. This invention provides all cis and trans isomeric forms.

According to the invention we also provide a process for the preparation of compounds of formula I which comprises:

a) reacting an amine of formula II:

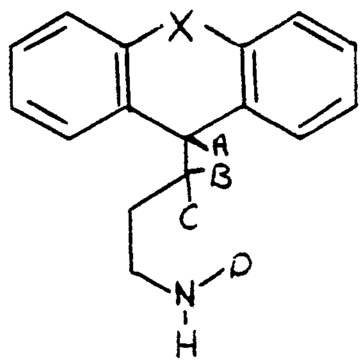

II

with an alkylation agent of formula III:

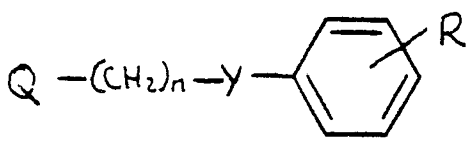

III

wherein Q is chlorine or bromine.

b) reducing an amide of formula IV:

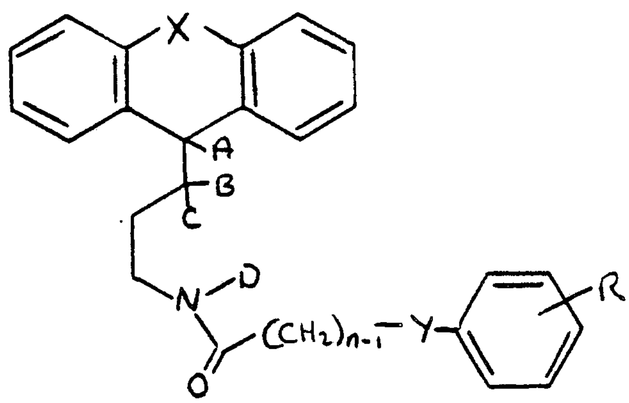

IV

c) producing a compound of formula I in which Y is -CH(OH)-, by reduction of the corresponding compound of formula I in which Y is -C( = O)-;

d) producing a compound of formula I in which R′ is H by hydrolysis of the corresponding compound of formula I in which R′ is C1 to 6 alkyl

e) producing a compound of formula I in which R′ is C1 to 6 alkyl by esterification of the corresponding compound of formula I in which R′ is H.

f) producing a compound of formula I in which R is $-C(CH_3)_2CH_2OH$, by reduction of the corresponding compound of formula I in which R is $-C(CH_3)_2COOR'$.

For process a), the reaction may be carried out in a suitable solvent, such as toluene or dimethylformamide, in the presence of a base, such as potassium bicarbonate or potassium carbonate, with or without a catalytic amount of potassium iodide.

For process b), the reduction of the amide may be carried out using a variety of reducing agents, for example lithium aluminium hydride, in a suitable solvent such as tetrahydrofuran.

The reduction of process c) may be carried out using a suitable reducing agent such as sodium borohydride, in an appropriate solvent, for example methanol or ethanol.

The hydrolysis of process d) may be carried out using an inorganic base, such as sodium hydroxide, in a lower alkanol solvent, such as ethanol or methanol.

The esterification of process e) may be carried out using standard esterification procedures, for example, by reacting the acid and the corresponding alcohol together in the presence of a dehydrating agent, such as dicyclohexylcarbodiimide, in a suitable solvent such as tetrahydrofuran.

For process f), a suitable reducing agent is lithium aluminium hydride, and the reaction may be carried out in an appropriate solvent, such as tetrahydrofuran.

The amides for process b) may be prepared from the corresponding amine of formula II and the corresponding carboxylic acid of formula V:

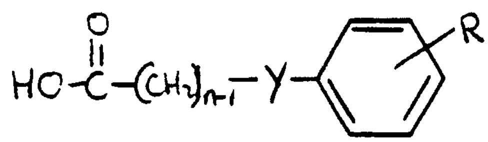

V

using standard acylation methods, for example by the reaction of a carboxylic acid of formula IV with dicyclohexylcarbodiimide and 1-hydroxysuccinimide in an inert solvent, such as tetrahydrofuran, to produce the corresponding N-hydroxysuccinimide ester, which is then reacted with an amine of formula II in an inert solvent, such as tetrahydrofuran or dimethylformamide or mixtures thereof to produce an amide, which can be reduced using a suitable reducing agent, such as lithium aluminium hydride, in an appropriate solvent, such as tetrahydrofuran or diethyl ether or mixtures thereof.

Compounds of the formulae III and V are known or can be made by known methods.

Many of the amines of formula II are known and may be prepared by suitable modification of the reported procedures. Amines of formula II in which C and D form a $CH_2CH_2$ chain may be prepared by the following method:

Ketones of formula VI:

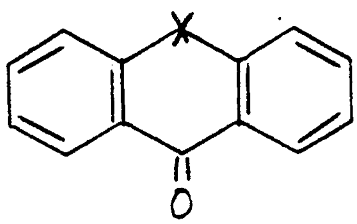

VI

are reacted with 4-lithiopyridine in a mixture of diethyl ether and tetramethylethylenediamine to provide the corresponding alcohols of formula VII:

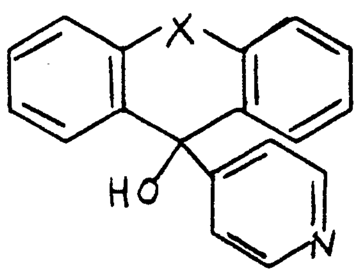

VII

The compounds of formula VII are reduced by catalytic hydrogenation in an appropriate solvent, such as acetic acid, with a suitable catalyst, such as platinum oxide, to provide the corresponding compounds of formula II.

Compounds of formula II where A is hydroxyl, B is hydrogen and X is as defined above may be dehydrated to the corresponding compounds of formula II where A and B taken together form an additional bond between the carbons to which they are attached. This dehydration may be accomplished with a suitable acid catalyst, such as para-toluenesulfonic acid, in an appropriate solvent such as chloroform.

The compounds of formula I are basic compounds and may be used as such or pharmaceutically acceptable acid addition salts may be prepared by treatment with various inorganic or organic acids, such as hydrochloric, hydrobromic, sulfuric, phosphoric, acetic, lactic, fumaric, malic, maleic, tartaric, citric, benzoic, methanesulfonic, or carbonic acid.

A particular subgroup of compounds of formula I which may be specifically mentioned is that in which X represents $-CH_2O-$, $-CH=CH-$ or $-CH_2CH_2-$; C and D together form a $CH_2CH_2$ chain; A represents hydroxy when B represents hydrogen, or A and B taken together form a second bond between the carbons to which they are attached; n is an integer between 3 and 6; Y represents $-C(=O)-$, $-CH_2-$, $-CH(OH)-$, -S-, -NH-, -O- or $-NHCH_2CH_2-$; and R represents H, tert butyl, $-C(CH_3)_2CH_2OH$ or $-C(CH_3)_2COOR'$ in which R′ represents C1 to 4 alkyl.

A further subgroup of compounds of formula I which may be specifically mentioned is that in which X represents $-OCH_2-$; A and B together form a second bond between the carbons to which they are attached; C represents hydrogen and D represents methyl, n is an integer between 3 and 5, Y represents $-CH_2-$, -CH-(OH)- or $-C(=O)-$; and R represents tert butyl.

Another subgroup of compounds of formula I which may be specifically mentioned is those in which X represents $-CH_2O-$ or $CH_2CH_2$, with the latter being preferred.

Alkyl groups that R, R′ and D may represent which may be specifically mentioned are methyl, ethyl, propyl, isopropyl, butyl, isobutyl, and tert butyl.

We prefer compounds of formula I in which R represents C1 to 6 alkyl, particularly tert-butyl. It is also preferred that the R group is in the 4 position on the benzene ring relative to the rest of the molecule.

We prefer compounds of formula I in which A and B form a second bond between the carbons to which they are attached.

We prefer compounds of formula I in which n represents 3, 4, or 5.

We prefer compounds of formula I in which Y represents -CH(OH)-, $-C(=O)-$ or $-CH_2-$, with -CH(OH)- being most preferred.

When C is hydrogen and D is C1 to 6 alkyl, we prefer that X is $-CH_2O-$, and that A and B together form a second bond between the carbons to which they are attached.

We prefer, however, compounds of formula I in which C and D form a $-CH_2CH_2-$ chain, in which case the most preferred compounds are those in which X is $-CH_2CH_2-$, Y is -CH(OH)-, and n is 3.

A particularly preferred compound of formula I is that in which X represents $-CH_2CH_2-$, A and B together form a second bond between the carbons to which they are attached, C and D form a saturated 2 carbon chain, Y represents -CH(OH)-, R represents tert butyl and n represents 3.

The compounds of formula I are useful as they possess pharmacological activity in animals, in particular they possess antihistaminic properties and show a low potential for sedation.

Antihistaminic activity is measured by the compound's ability to inhibit the wheal response to histamine in a rat dermal vascular permeability test. Groups of 10 male rats are administered the test compound orally one hour prior to an intravenous injection of 1 ml of a 0.5% Evans Blue dye into naive animals. Ten minutes later the animals are challenged by intradermal injection of 0.1 ml of solutions of histamine at 10 µg, 2 µg, 1 µg 0.5 µg per 0.1 ml into separate sites on the back. Five minutes following the histamine injections the animals are killed, the skin reflected and the mean diameter of the three wheals determined. The percent inhibition is calculated as the difference in mean diameter between the control and the drug treated group divided by the control diameter times 100. Compounds of the formula I were active in inhibiting the wheal response due to histamine at oral doses of 1-25 mg/kg. In particular, the compound of Example 4 caused a 50% inhibition of the wheal response to the 0.5 µg histamine challenge at a dose of about 3 mg/kg.

The sedative effects of the compounds are observed by behavioral observation of groups of mice or rats. Sedative effects were generally not observed for oral doses of the compounds of formula I of 200 mg/kg or less.

Some of the compounds of this invention possess a long duration of action of antihistaminic activity. For example the compound of Example 2 at an oral dose of 2 mg/kg caused a 70% inhibition of the 0.5 µg histamine wheal response after 6 hours.

The compounds of the invention are indicated for use in the treatment of diseases and conditions mediated by the response of H receptors to histamine, eg allergy related diseases and conditions. Thus, according to a further aspect of the invention there is provided a method of treatment of a disease or condition mediated by the response of $-H_1$ receptors to histamine which comprises administration of a therapeutically effective quantity of a compound of formula I to an animal or human patient suffering from such a disease or condition. Conditions which may be specifically mentioned are: pollinosis; urticaria; allergic rhinitis; seasonal rhinitis; conjunctivitis; hayfever etc.

For the above mentioned uses the doses administered will, of course, vary with the compound

employed, the mode of administration and the treatment desired. However, in general, satisfactory results are obtained when the compound is administered at a daily dosage of from about 0.1mg to about 20mg per kg of animal body weight, preferably given in divided doses 1 to 4 times a day or in sustained release form. For man the total daily dose is in the range of from 7.0mg to 1,400mg and unit dosage forms suitable for oral administration comprise from 2.0mg to 1,400mg of the compound admixed with a solid or liquid pharmaceutical diluent or carrier.

The compounds of formula I may be used on their own or in the form of appropriate medicinal preparations for enteral, parenteral or topical administration.

According to the invention there is also provided a pharmaceutical composition comprising preferably less than 80% and more preferably less than 50% by weight of a compound of formula I, or a pharmaceutically acceptable derivative thereof, in admixture with a pharmaceutically acceptable adjuvant, diluent or carrier.

Examples of such adjuvants, diluents and carriers are:
for tablets and dragees - lactose, starch, talc, stearic acid;
for capsules - tartaric acid or lactose; for injectable solutions - water, alcohols, glycerin, vegatable oils;

Compositions in a form suitable for oesophageal administration include tablets, capsules and dragees; compositions in a form suitable for administration to the skin include creams, eg oil-in-water emulsions or water-in-oil emulsions;
compositions in a form suitable for administration to the eye include drops amd ointments.

According to another aspect of the invention, we provide a process for the manufacture of a medicament containing a compound of formula I as an active ingredient for use in the treatment of a human or animal patient suffering from a disease or ccndition mediated by the response of $H_1$ receptors to histamine.

The compounds of formula I have the advantage that they are less toxic, more efficacious, are longer acting, have a broader range of activity, are more potent, show reduced sedation effects, produce fewer other CNS side effects, are more easily absorbed or have other useful pharmacological properties, than compounds of similar structure.

The following non-limiting intermediates and examples are provided to exemplify the preparation of the compounds of formula I.

Intermediate 1

10,11-Dihydro-5-(4-pyridinyl)-5H-dibenzo[a,d]cyclohepten-5-ol

To a stirred solution of 4-bromopyridine (37.9 g, 0.24 mol) in anhydrous ether (475 ml) at -75 °C under a nitrogen atmosphere was added n-butyllithium (160 ml of a 1.5 M hexane solution, 0.24 mol). Tetramethylethylenediamine (36.2 ml, 0.24 mol) was added and the solution was stirred at -75 °C for 1 hour. A solution of dibenzosuberone (50.1 g, 0.24 mol) in anhydrous ether (250 ml) was added dropwise, the reaction mixture was stirred at -75 °C for 40 minutes, warmed to ambient temperature and stirred at that temperature for 1.5 hours. Water (650 ml) was added dropwise. The solid which crystallized was isolated by filtration to give 43.54 g of 10,11-dihydro-5-(4-pyridinyl)-5H-dibenzo[a,d]cyclohepten-5-ol, mp 174-177 °C. Recrystallization from ethyl acetate-hexane gave material of mp 183-185 °C.

Intermediate 2

10,11-Dihydro-5-(4-piperidinyl)-5H-dibenzo-[a,d]cyclohepten-5-ol acetic acid (1:1) salt

To a solution of 10,11-dihydro-5-(4-pyridinyl)-5H-di benzo[a,d]cyclohepten-5-ol (44.6 g, 0.155 mol) in acetic acid (700 ml) was added platinum oxide (3.5 g) and the mixture was shaken on a Parr apparatus at 55-60 °C at 45 psi of hydrogen for 16 hours. An additional portion of platinum oxide (1.5 g) was added and the mixture was shaken at 55-60 °C at 45 psi hydrogen for an additional 20 hours. The catalyst was removed by filtration and the majority of the solvent evaporated to give an oil. The above oil was crystallized from ethyl ether (600 ml) and acetone (80 ml) to give 28.8 g of ]O,11-dihydro-5-(4-piperidinyl)-5H-dibenzo[a,d]cyclohepten-5-ol acetic acid (1:1) salt, mp 89-92 °C. Recrystallization of the free base from

ethanol gave material of mp 217-219 °C.

Intermediate 3

4-(10,11-Dihydro-5H-dibenzo[a,d]cyclohepten-5-ylidene)piperidine hydrochloride

To a stirred solution of lO,ll-dihydro-5-(4-piperidinyl)-5H-dibenzo[a,d]cyclohepten-5-ol acetic acid (1:1) salt (30.35 g, 0.086 mol) in chloroform (460 ml) was added p-toluenesulfonic acid monohydrate (30.38 g, 0.160 mol) and the mixture was heated to reflux under nitrogen for 4 hours. The reaction was cooled to ambient temperature, 5% NaOH (200 ml) added, and extracted with chloroform (3 x 100 ml). The combined chloroform extracts were washed with saturated NaCl (200 ml), dried over magnesium sulfate, and the solvent removed to give 24.6 g of a yellow solid. The above solid was dissolved in methanol (100 ml) and 2-propanol (150 ml), acidified with HCl gas, and the solid which formed was isolated by filtration to give 19.6 g of 4-(10,11-dihydro-5H-dibenzo[a,d]cyclohepten-5-ylidene)-piperidine hydrochloride, mp >310 °C.

Intermediate 4

6,11-Dihydro-11-(4-pyridinyl)dibenz[b,e]-oxeoin-11-ol

Prepared by the method used in Intermediate 1, using dibenz[b,e]oxepin-11(6H)-one. Mp 208-209 °C (ethanol).

Intermediate 5

6,11-Dihydro-11-(4-piperidinyl)dibenz[b,e]-oxepin-11-ol acetic acid (1:1) salt

Prepared by the method of Intermediate 3, using 6,11-dihydro-11-(4-pyridinyl)dibenz[b,e]oxepin-11-ol. Mp 243-245 °C.

Intermediate 6

4-(Dibenz[b,e]oxepin-11(6H)-ylidene)-piperidine hydrochloride

Prepared by the method for Intermediate 3, using 6,11-dihydro-11-(4-piperidinyl)dibenz[b,e]oxepin-11-ol acetic acid salt. Mp >300 °C (2-propanol).

Intermediate 7

6-[(2-Phenylethyl)amino]-6-oxohexanoic acid

To a stirred solution of (2-phenylethyl)amine (6.9 g, 0.06 mol) in dichloromethane (100 ml), at ambient temperature under a nitrogen atmosphere, were added triethylamine (17.2 g, 0.17 mol) and a solution of ethyl 5-(chloroformyl)pentanoate (11.02 g, 0.057 mol) in dichloromethane (50 ml). The reaction was stirred for 18 hours at ambient temperature. The reaction was washed with 1N HCl (2 x 100 ml), 2N sodium bicarbonate (2 x 100 ml), saturated NaCl, dried over magnesium sulfate and the solvent evaporated to give 16.3 g of an oil. To a stirred solution of the above oil in methanol (200 ml) was added 10% NaOH (16 ml) and the reaction was heated to reflux for 1 hour. The reaction was poured onto 10% HCl (500 ml) and extracted with chloroform (3 x 200 ml). The combined chloroform extracts were dried over magnesium

sulfate and evaporated to give 12.1 g of a solid. This solid was recrystallized from ethyl acetate and hexane to provide 10.0 g of 6-[(2-phenylethyl)amino]-6-oxohexanoic acid, mp 110-111 C.

Intermediate 8

3-[4-(1,1-Dimethylethyl)phenoxy]-1-chloropropane

To a stirred solution of 4-tert-butylphenol (20.0 g, 0.16 mol) in acetone (500 ml) were added potassium carbonate (100 g, 0.72 mol) and 1-bromo-3-chloropropane (50.6 g, 0.33 mol). The reaction was heated to 50°C for 18 hours. The reaction was filtered and the solvents evaporated to provide 30.3 g of 3-[4-(1,1-dimethylethyl)phenoxy]-1-chloropropane as a colorless oil; 200 MHz NMR 1.47 (s, 9H), 3.36 (p, J=5.5Hz, 2H), 3.88 (t, J=5.5Hz, 2H), 4.22 (t, J=5.5Hz, 2H), 7.0, 7.47 (ABq, J=9.6Hz, 4H).

Intermediate 9

1-[4-(1,1-Dimethylethyl)phenyl]-4-chlorobutanone

This compound was prepared by suitable modification of the procedure described by Westingh, C. van der; Hermans, B.; Raeymaekers, F.; Eycken, C. van der Ind. Chim-Belge., 1960, 25, 1073 as follows. To a stirred solution of 4-chlorobutyryl chloride (72.4 ml, 0.647 mol) in dichloromethane (1 l) at 5°C under nitrogen was added aluminum chloride (94.6 g, 0.71 mol) and the mixture was stirred for 1 hour. To this mixture was added dropwise a solution of t-butylbenzene (100 ml, 0.647 mol) in dichloromethane (100 ml). The reaction was warmed to ambient temperature and stirred at that temperature overnight. The reaction was poured onto a mixture of ice (1 l) and 0.5N HCl (1 l). The phases were separated and the aqueous phase was extracted with dichloromethane (2 x 300 ml). The combined dichloromethane extracts were washed with 5% NaOH (2 x 300 ml), saturated NaCl (250 ml), dried over magnesium sulfate and the solvent removed to give 149.8 g of an oil. This oil was crystallized from hexanes (150 ml) to provide 118.3 g of 1-[4-(1,1-dimethylethyl)phenyl]-4-Chlorobutanone, mp 46-49°C.

Intermediate 10

4-[4-(1,1-Dimethylethyl)phenyl]butanoic acid

This compound was prepared by suitable modification of the procedures described by Martin, E. L., J. Am. Chem. Soc., 1936, 58, 1841 as follows. To a stirred suspension of aluminium chloride (266 g, 2.0 mol) in dichloromethane (1 l) cooled in an ice water bath under a nitrogen atmosphere was added succinic anhydride (100 g, 1.0 mol). The mixture was warmed to ambient temperature and a solution of t-butylbenzene (122.4 g, 0.897 mol) in dichloromethane was added dropwise. The reaction was stirred at ambient temperature for 16 hours. The reaction was poured onto 2N HCl and extracted with chloroform (2 x 800 ml). The combined organic extracts were washed with saturated NaCl, dried over magnesium sulfate and the solvent evaporated to give 156.6 g of a tan solid. Recrystallization from toluene (700 ml) and hexanes (200 ml) gave 94.5 g of 4-[4-(1,1-dimethylethyl)phenyl]-4- oxobutanoic acid, mp.112-114°C.

To a stirred suspension of zinc amalgam (180 g) in water (150 ml) were carefully added conc. HCl (325 ml), toluene (150 ml), acetic acid (40 ml), and 4-[4-(1,1-dimethylethyl)phenyl]-4-oxobutanoic acid (45 g, 0.19 mol). The reaction was heated to reflux for 24 hours. The reaction was cooled to ambient temperature, the solution decanted and extracted with ether (3 x 300 ml). The combined ether extracts were washed with saturated sodium ch]oride (300 ml), dried over magnesium sulfate, and concentrated to give 42.2 g of an off-white solid. Recrystallization from hexanes gave 25.0 g of 4-[4-(1,1-dimethylethyl)phenyl]butanoic acid, mp 55-57°C.

Intermediate 11

4-[4-(1,1-Dimethylethyl)phenyl]-1-bromobutane

To a stirred suspension of lithium aluminium hydride (4.0 g, 0.105 mol) in THF (100 ml) at 0° C under a nitrogen atmosphere was added a solution of 4-[4-(1,1-dimethylethyl)phenyl]butanoic acid (10.31 g, 0.0455 mol) in THF (80 ml). The reaction was heated to reflux for 6 hours. The reaction was cooled to 0° C and water (4 ml), 15% NaOH (4 ml) and water (12 ml) were carefully added. Ethyl ether (150 ml) was added, the mixture warmed to ambient temperature, and the precipitated salts were removed by filtration. Removal of solvent gave 9.15 g of 4-[4-(1,1-dimethylethyl)phenyl]butanol as a colorless oil.

To a stirred solution of phosphorous tribromide (1.75 ml, 0.185 mol) in benzene (100 ml) at 8° C was added a solution of 4-[4-(1,1-dimethylethyl)phenyl]butanol (9.15 g, 0.0444 mo[) in benzene (50 ml). The solution was stirred at 10° C for 2 hours, water (200 ml) was added, and the mixture was extracted with ethyl ether (2 x 150 ml). The combined ether extracts were washed with water (2 x 150 ml), saturatcd NaCl (150 ml) and dried over magnesium sulfate. Removal of solvent gave 11.6 g of an oil. This oil was purified by silica gel chromatography, eluting with 5% ethyl acetate-hexane, to give 3.16 g of 4-[4-(1,1-dimethylethyl)phenyl]-1-bromobutane as a colorless oil, NMR δ 1.37 (s, 9H), 1.8 (m, 2H), 2.45 (t, J=7Hz, 2H), 3.38 (t, J=7Hz, 2H). 7.1, 7.3 (ABq, J=9.5Hz, 4H).

Intermediate 12

3-Dibenz[b,e]oxeoin-11(6H)-ylidene-N-methyl-1-propanamine hydrochloride

This compound was prepared by suitable modification of the procedure described by Bickelhaupt, F., Stach, K., Thiel, M., Monatsh., 1964, 95, 485 as follows. To a stirred solution of ethyl chloroformate (188 ml, 1.97 mol) in toluene (200 ml) at 85° C under nitrogen was added a solution of 3-dibenz[b,e]oxepin-11(6H)-ylidene-N,N-dimethyl-1-propanamine (184.6 g, 0.658 mol) in toluene (350 ml) and the mixture was stirred at that temperature for 6 hours. The solvents were evaporated and the residual oil was dissolved in toluene (1 l) and washed with 10% HCl (3 x 100 ml). The toluene so]ution was dried over magnesium sulfate and the solvent evaporated to give 166.8 g of an oil. To a stirred solution of this oil in 95% ethanol (525 ml) was added potassium hydroxide (137.7 g, 246 mol) and the solution was heated to reflux under nitrogen for 24 hours. The reaction was cooled to ambient temperature, poured into water (3 l), acidified with conc. HCl and washed with toluene (4 x 500 ml). The aqueous layer was basified with 50% sodium hydroxide and extracted with chloroform (3 x 1 l). The combined chloroform extracts were dried over magnesium sulfate and the solvent evaporated to give 103 g of an oil. The above oil was dissolved in methanol (200 ml) and ethyl acetate (300 ml) and acidified with HCl gas. The solid which formed was isolated by filtration to give 99.1 g of 3-dibenz[b,e]oxepin-11(6H)-ylidene-N-methyl-1-propanamine (5:1 mixture of E:Z isomers), mp 236-237° C.

A 20.0 g sample of the above product was recrystallized twice from 2-propanol, and vacuum dried at 75° C for 72 hours to provide 12.2 g of E-3-dibenz[b,e]oxepin-11(6H)-ylidene-Nmethyl-1-propanamine, mp 240-242° C.

Example 1

4-[4-(10,11-Dihydro-5H-dibenzo[a,d]cyclohepten-5-ylidene)-1-piperidinyl]-1-[4-(1,1-dimethylethyl)phenyl]-1-butanone maleate

To a stirred solution of 4-(10,11-dihydro-5H-dibenzo[a,d]cyclohepten-5-ylidene)piperidine (71.3 g, 0.249 mol) in toluene (1 l) were added 4′-tert-butyl-4-chlorobutyrophenone (91.5 g, 0.38 mol), potassium bicarbonate (53 g, 0.5 mol) and potassium iodide (2.5 g, 0.015 mol). The mixture was heated to reflux under a nitrogen atmosphere for 48 hours. The mixture was cooled to ambient temperature, poured into water (2 l), the phases separated, and the aqueous phase was extracted with chloroform (2 x 500 ml). The combined organic extracts were dried over magnesium sulfate and the solvent removed to give 172 g of an oil. The oil was dissolved in hot ethyl acetate (500 ml) and treated with maleic acid (74 g, 0.63 mol) in hot ethyl acetate (300 ml); upon cooling, a white solid crystallized and was collected by filtration to give 165.3 g. This solid was recrystallized from ethanol (800 ml) to give 110.6 g of 4-[4-(10,11-dihydro-5H-dibenzo[a,d]cyclohepten-5-ylidene)-1-piperidinyl]-1-[4-(1,1-dimethylethyl)phenyl]-1-butanone maleate, mp 178-180 °C.

Example 2

4-(10,11-Dihydro-5H-dibenzo[a,d]cyclohepten-5-ylidene)-1-[4-[4-(1,1-dimethylethyl)phenyl]-4-hydroxybutyl]-piperidine

To a stirred solution of 4-[4-(10,11-dihydro-5H-dibenzo[a,d]cyclohepten-5-ylidene)-1-piperidinyl]-1-[4-(1,1-dimethylethyl)phenyl]-1-butanone maleate (110.6 g, 0.22 mol) in chloroform (750 ml) and water (1 l) was added enough 15% NaOH to give a solution of pH 11. The phases were separated and the aqueous phase extracted with $CHCl_3$ (3 x 500 ml). The combined chloroform extracts were dried over $MgSO_4$ and the solvent removed to give 96.1 g of an oil. To a stirred solution of the above oil in methanol (2 l) at 0 °C under nitrogen was added sodium borohydride (29.7 g, 0.79 mol). The reaction was allowed to warm to ambient temperature and stirred at that temperature overnight. Acetone (200 ml) was added to the reaction dropwise, and the solvents were removed. The solid residue was dissolved in water (2 l) and extracted with chloroform (3 x 750 ml). The combined chloroform extracts were dried over magnesium sulfate and the solvent removed to provide 128 g of a white solid. This solid was recrystallized from 2-propanol (750 ml) and then from 2-propanol (500 ml) and methanol (200 ml), and vacuum dried at 85 °C for 5 days to provide 64.5 g of 4-(10,11-dihydro-5H-dibenzo[a,d]cyclohepten-5-ylidene)-1-[4-[4-(1,1-dimethylethyl)phenyl]-4-hydroxybutyl]piperidine, mp 157-158 °C.

Example 3

4-(10,11-Dihydro-5H-dibenzo[a,d]cyclohepten-5-ylidene)-1-[4-[4-(1,1-dimethylethyl)phenyl]butyl]piperidine hydrochloride

To a stirred solution of 4-[4-(1,1-dimethylethyl)phenyl]butanoic acid (7.26 g, 0.033 mol) in tetrahydrofuran (145 ml) were added N-hydroxysuccinimide (3.80 g, 0.003 mol) and dicyclohexylcarbodiimide (6.81 g, 0.033 mol). The reaction was stirred at ambient temperature under nitrogcn for 21 hours. The precipitated solid was removed by filtration. To a stirred solution of the filtrate, at ambient temperature under nitrogen was added a solution of 4-(10,11-dihydro-5H-dibenzo[a,d]cyclohepten-5-ylidene)piperidine (9.16 g, 0.032 mol) in tetrahydrofuran (130 ml) and dimethylformamide (150 ml), and the reaction was stirred at ambient temperature for 24 hours. The solvents were removed and the residue was dissolved in chloroform (250 ml). The chloroform solution was washed with 5% NaOH (2 x 150 ml), saturated NaCl (200 ml). dried over $MgSO_4$, and the solvent removed to provide 19.4 g of an oil. This oil was purified by silica gel chromatography on a Waters Prep 500 HPLC, eluting with ammoniated 0.5% methanol-chloroform to provide 9.80 g of 4-(10,11-dihydro-5H-dibenzo[a,d]cyclohepten-5-ylidene)-1-[4-[4-(l,l-dimethyl ethyl)phenyl]-1-oxobutyl]piperidine as an oil.

To a stirred solution of the above oil in anhydrous ether (500 ml) at 0 °C under nitrogen was added lithium aluminum hydride (4.18 g, 0.11 mol). The reaction was warmed to ambient temperature and stirred at that temperature for 20 hours. The reaction was cooled to 0 °C and water (4 ml), 15% NaOH (4 ml) and water (12 ml) were carefully added. The insoluble salts were removed by filtration through celite, and the filtrate was concentrated to an oil. The above oil was dissolved in chloroform (250 ml), washed with water (250 ml), saturated NaCl (150 ml), dried over $MgSO_4$, and the solvent removed to give 9.95 g of an oil. The above oil was dissolved in ethyl acetate (30 ml) and acidified with HCl gas, and ether (75 ml) was added. The solid was collected by filtration, recrystallized from ethyl acetate (75 ml), cyclohexane (50 ml) and methanol (5 ml), and vacuum dried at 80 °C for 9 days to provide 4.98 g of 4-(10,11-dihydro-5H-dibenzo-[a,d]cyclohepten-5-ylidene)-1-[4-[4-(1,1-dimethylethyl)phenyl]butyl]piperidine hydrochloride, mp 177-179 °C.

Example 4

4-Dibenz[b,e]oxepin-11(6H)-ylidene-1-[4-[4-(1,1-dimethylethyl)phenyl]butyl]piperidine hydrochloride

To a stirred solution of 4-dibenz[b,e]oxepin-11(6H)- ylidenepiperidine (7.1 g, 0.026 mol) in dimethylformamide (70 ml) were added potassium bicarbonate (5.12 g, 0.051 mol) and a solution of 4-[4-(1,1-dimethylethyl)phenyl)-1-bromobutane (7.6 g, 0.028 mol) in dimethylformamide (30 ml). The reaction was heated to 70° C for 36 hours under a nitrogen atmosphere. The reaction mixture was cooled to ambient temperature, poured into water (300 ml) and extracted with ethyl acetate (2 x 200 ml). The combined ethyl acetate extracts were washed with water (3 x 150 ml), saturated NaCl, and dried over $MgSO_4$. Removal of solvent gave 12.67 g of an oil. This oil was dissolved in ether (300 ml) and ethanol (50 ml) and acidified with HCl. The solid which formed was isolated by filtration, and vacuum dried at 80° C for 72 hours to provide 7.61 g of 4-dibenz[b,e]oxepin-11(6H)-ylidene-1-[4-[4-(1,1-dimethylethyl)phenyl]butyl]piperidine hydrochloride, mp 218-219° C.

Example 5

4-[4-(10,11-Dihydro-5-hydroxy-5H-dibenzo[a,d]cyclohepten-5-yl)-1-piperidinyl]-1-[4-(1,1-dimethylethyl)-phenyl]-1-butanone

Prepared by the method of Example 1, using 10,11-dihydro-5-(4-piperidinyl)-5H-dibenzo[a,d]-cyclohepten-5-ol. Mp 132-134° C (ethanol).

Example 6

4-(4-Dibenz[b,e]oxepin-11(6H)-ylidene-1-piperidinyl)-1-[4-(1 ,1-dimethylethyl)phenyl]-1-butanone hydrochloride

Prepared by the method of Example 1, using 4-dibenz[b,e]oxepin-11(6H)-ylidenepiperidine. Mp 203-204° C (2-propanol).

Example 7

4-Dibenz[b,e]oxepin-11(6H)-ylidene-1-[4-[4-(1,1-dimethylethyl)phenyl]-4-hydroxybutyl]piperidine

Prepared by the method of Example 2, using 4-[4-(dibenz[b,e]oxepin-11(6H)-ylidene)-1-piperidinyl]-1-[4-(1,1-dimethylethyl)-phenyl]-1-butanone hydrochloride. Mp 159.5-161° C (2-propanol).

Example 8

4-[4-(6,1]-Dihydro-[1-hydroxydibenz[b,e]oxepin-11-yl)-1-piperidinyl]-1-[4-(1,1-dimethylethyl)phenyl]butanone

Prepared by the method of Example 1, using 6,11-dihydro-11- 4-piperidinyl)dibenz[b,e]oxepin-11-ol. Mp 143-145° C (2-propanol).

Example 9

11-[1-[4-[4-(1,1-Dimethylethyl)phenyl]-4-hydroxybutyl]-4-piperidinyl]-6,11-dihydrodibenz[b,e]oxepin-11-ol

Prepared by the method of Example 2, using 4-[4-(6,11-dihydro-11-hydroxydibenz[b,e]oxepin-11-yl)-1-piperidinyl]-1-[4-(1,1-dimethylethyl)phenyl]butanone. Mp 135-137° C and 162-165° C (ether).

Example 10

11-[1-[4-[4-(1,1-Dimethylethyl)phenyl]butyl]-4-piperidinyl]-6,11-dihydrodibenz[b,e]oxepin-11-ol

Prepared by the method of Example 4, using 6,11-dihydro-11-(4-piperidinyl)dibenz[b,e]oxepin-11-ol. Mp 140-141° C (2-propanol).

Example 11

10,11-Dihydro-5-[1-[4-[4-(1,1-dimethylethyl)phenyl]-4-hydroxybutyl]-4-piperidinyl]-5H-dibenzo[a,d]-cyclohepten-5-ol

Prepared by the method of Example 2, using 4-[4-(10,11-dihydro-5-hydroxy-5H-dibenzo[a,d]-cyclohepten-5-yl)-1-piperidinyl]-1-[4-(1,1-dimethylethyl)phenyl]-1-butanone. Mp 142-144° C (ethanol-water).

Example 12

10,11-Dihydro-5-[1-[4-[4-(1,1-dimethylethyl)phenyl]butyl]-4-piperidinyl]-5H-dibenzo[a,d]cyclohepten-5-ol

Prepared by the method of Example 4, using 10,11-dihydro-5-(4-piperidinyl)-5H-dibenzo[a,d]-cyclohepten-5 -ol. Mp 99-101° C (2-propanol).

Example 13

4-[4-(5H-Dibenz[a,d]cyclohepten-5-ylidene)-1-piperidinyl]-1-[4-(1,1-dimethylethyl)phenyl]-1-butanone maleate

Prepared by the method of Example 1, using 4-(5H-dibenzo[a,d]cyclo- hepten-5-ylidene)piperidine. Mp 161-162° C (2-propanol).

Example 14

4-(5H-Dibenzo[a,d]cyclohepten-5-ylidene)-1-[4-[4-(1,1-dimethylethyl)phenyl]-4-hydroxybutyl]piperidine

Prepared by the method of Example 2, using 4-(5H-dibenzo[a,d]cyclohepten-5-ylidene)-1-piperidinyl]-1-[4-(1,1-dimethylethyl)-phenyl]-1-butanone maleate. Mp 141-142° C (2-propanol).

Example 15

5-[4-(10,11-Dihydro-5H-dibenzo[a,d]cyclohepten-5-ylidene)-1-piperidinyl]-1-[4-(1,1-dimethylethyl)phenyl]-1-pentanone maleate

Prepared by the method of Example 1, using 4′-tert-butyl-5-chloro-valerophenone. Mp 140-141° C (2-propanol, ether).

Example 16

6-[4-(10,11-Dihydro-5H-dibenzo[a,d]cyclohepten-5-ylidene)-1-piperidinyl]-1-[4-(1,1-dimethylethyl)phenyl]-1-hexanone fumarate

Prepared by the method of Example 2, using 4′-tert-butyl-6-bromo-hexanophenone. Mp 185-187 °C (ethyl acetate, ether).

Example 17

4-(10,11-Dihydro-5H-dibenzo[a,d]cyclohepten-5-ylidene)-1-[5-[4-(1,1-dimethylethyl)phenyl]-5-hydroxypentyl]-piperidine

Prepared by the method of Example 2, using 5-[4-(10,11-dihydro-5H-dibenzo[a,d]cyclohepten-5-ylidene)-1-piperidinyl]-1-[4-(1,1-dimethylethyl)phenyl]-1-pentanone maleate. Mp 157-158 °C (2-propanol).

Example 18

4-(10,11-Dihydro-5H-dibenzo[a,d]cyclohepten-5-ylidene)-1-[6-[4-(1,1-dimethylethyl)phenyl]-6-hydroxyhexyl]-piperidine fumarate

Prepared by the method of Example 2, using 6-[4-(10,11-dihydro-5H-dibenzo[a,d]cyclohepten-5-ylidene)-1-piperidinyl]-1-[4-(1,1-dimethylethyl)phenyl]-1-hexanone fumarate. Mp 120-121 °C (2-propanol, ethyl acetate).

Example 19

4-(10,11-Dihydro-5H-dibenzo[a,d]cyclohepten-5-ylidene)-1-[5-[4-(1,1-dimethylethyl)phenyl]pentyl]piperidine fumarate

Prepared by the method of Example 4, using 4-(10,11-dihydro-5H-dibenzo[a,d]cyclohepten-5-ylidene) piperidine. Mp 157-158 °C

Example 20

4-(10,11-Dihydro-5H-dibenzo[a,d]cyclohepten-5-ylidene)-1-[6-[4-(1,1-dimethylethyl)phenyl]hexyl]piperidine maleate

Prepared by the method of Example 3, using 6-[4-(1,1-dimethylethyl)phenyl]hexanoic acid. Mp 166-167 °C (ethyl acetate).

Example 21

4-(10,11-Dihydro-5H-dibenzo[a,d]cyclohepten-5-ylidene)-1-[3-[4-(1,1-dimethylethyl)phenoxy]propyl]-piperidine hydrochloride

To a stirred solution of 4-(10,11-dihydro-5H-dibenzo[a,d]cyclohepten-5-ylidene)piperidine (8.0 g, 0.029 mol) in dimethylformamide (200 ml) were added potassium carbonate (100 g, 0.72 mol) and 3-[4-(1,1-dimethylethyl)phenoxy]-l-chloropropane (9.0 g, 0.043 mol). The reaction mixture was heated to 60-65 °C for 18 hours. The reaction was poured into water (1 l) and extracted with ethyl acetate (3 x 300 ml). The

combined ethyl acetate extracts were washed with water (3 x 200 ml), saturated with NaCl (200 ml), dried over magnesium sulfate, and the solvent removed to give 16.1 g of an oil. This oil was purified by silica gel chromatography on a Waters Prep 500 HPLC, eluting with ammoniated 25% ethy] acetate-hexane, to give 5.0 g of an oil. This oil was dissolved in ethyl acetate (75 ml) and 2-propanol (5 ml) and acidified with HCl gas. The solid which formed was collected by filtration, and vacuum dried at 90°C for 5 days to provide 3.1 g of 4-(10,11-dihydro-5H-dibenzo[a,d]cyclohepten-5-ylidene)-1-[3-[4-(1,1-dimethylethyl)phenoxy]propyl]-piperidine hydrochloride, mp 209-210°C.

Example 22

4-(10,11-Dihydro-5H-dibenzo[a,d]cyclohepten-5-ylidene)-1-[3-[[4-(1,1-dimethylethyl)phenyl]thio]propyl]-piperidine fumarate

Prepared by the method of Example 21, using 3-[[4-(1,1-dimethylethyl)phenyl]thio]-1-chloropropane. Mp 189-190°C (ethyl acetate, 2-propanol).

Example 23

3-[4-(10,11-Dihydro-5H-dibenzo[a,d]cyclohepten-5-ylidene)-1-piperidinyl]-N-[4-(1,1-dimethylethyl)phenyl]-propanamine hydrochloride

Prepared by the method of Example 4, using 4-(10,11-dihydro-5H-dibenzo[a,d]cyclohepten-5-ylidene) piperidine for 4-dibenz[b,e]oxepin-11(6H)-ylidenepiperidine and N-(3-chloropropyl)-4-(1.,1-dimethylethyl)-benzeneamine. Mp 217-220°C (ethyl acetate, methanol).

Example 24

4-[4-[4-(10,11-Dihydro-5H-dibenzo[a,d]cyclohepten-5-ylidene)-1-piperidinyl]-1-oxobutyl]-α,α-dimethylbenzeneacetic acid ethyl ester hydrobromide

Prepared by the method of Example 1, using 4-(4-chloro-1-oxobutyl)-α,α-dimethylbenzeneacetic acid ethyl ester. Mp 187-189°C (methanol).

Example 25

4-[4-[4-(10,11-Dihydro-5H-dibenzo[a,d]cyclohepten-5-ylidene)-1-piperidinyl]-1-hydroxybutyl]-α,α-dimethylbenzeneacetic acid ethyl ester

Prepared by the method of Example 2, using 4-[4-[4-(10,11-dihydro-5H-dibenzo[a,d]cyclohepten-5-ylidene) -1-piperidinyl]-1-oxobutyl]-α,α-dimethylbenzeneacetic acid ethyl ester hydrobromide. Mp 142-144°C (ethanol-water).

Example 26

4-[4-[4-(10,11-Dihydro-5H-dibenzo[a,d]cyclohepten-5-ylidene)-1-piperidinyl]-1-hydroxybutyl]-α,α-dimethylbenzeneacetic acid

To a stirred suspension of 4-[4-[4-(10,11-dihydro-5H-dibenzo[a,d]cyclohepten-5-ylidene)-1-piperidinyl]-1-hydroxybutyl]-α,α-dimethylbenzeneacetic acid ethyl ester (5.62 g, 0.010 mol) in ethanol (150 ml) was added 15% NaOH (15 ml) and the mixture was heated to reflux, under nitrogen, for 2 hours. The reaction was cooled to ambient temperature, concentrated to approximately ½ its original volume, dissolved in water (150 ml), IN HCl was added to give a solution of pH 7, and extracted with chloroform (3 x 150 ml). the combined chloroform extracts were washed with saturated NaCl, dried over $MgSO_4$, and the solvent evaporated to give 5.7 g of a solid. This solid was recrystallized from absolute ethanol (75 ml), and vacuum dried at 50°C for 3 days to give 2.59 g of 4-[4-[4-(10,11-dihydro-5H-dibenzo[a,d]cyclohepten-5-ylidene)-1-piperidinyl]-1-hydroxybutyl]α,α-dimethylbenzeneacetic acid, mp 135-137°C.

Example 27

4-[4-[4-(10,11-Dihydro-5H-dibenzo[a,d]cyclohepten-5-ylidene)-1-piperidinyl]-1-oxobutyl]-α,α-dimethylbenzeneacetic acid

Prepared by the method of Example 26, using 4-[4-[4-(10,11-dihydro-5H-dibenzo[a,d]cyclohepten-5-ylidene)-1-piperidinyl]-1-oxobutyl]-α,α-dimethylbenzeneacetic acid ethyl ester hydrobromide. Mp 122-125°C (ethanol).

Example 28

4-(10,11-Dihydro-5H-dibenzo[a,d]cyclohepten-5-ylidene)-1-[4-[4-(1,1-dimethyl-2-hydroxyethyl)phenyl]-4-hydroxybutyl] piperidine

To a stirred suspension of lithium aluminium hydride (1.16 g, 0.0305 mol) in tetrahydrofuran (150 ml) at 0°C under nitrogen, was added dropwise a solution of 4-[4-[4(10,11-dihydro-5H-dibenzo[a,d]cyclohepten-5-ylidene)-1-piperidinyl]-1-hydroxybutyl]-α,α-dimethylbenzeneacetic acid ethyl ester (8.2 g, 0.0153 mol) in tetrahydrofuran (80 ml). The reaction mixture was stirred at ambient temperature overnight and cooled to 0°C. Water (2.2 ml), 15% NaOH (2.2 ml) and water (6.6 ml) were carefully added. The mixture was warmed to ambient temperature, filtered through celite, and the solvent evaporated to give 7.8 g of a solid. This solid was recrystallized from 2-propanol and vacuum dried at 50°C for 60 hours to provide 4.11 g of 4-(10,11-dihydro-5H-dibenzo[a,d]cyclohepten-5-ylidene)-1-[4-[4-(1,1-dimethyl-2-hydroxyethyl)phenyl]-4-hydroxybutyl]piperidine, mp 157-159°C.

Example 29

6-[4-(10,11-Dihydro-5H-dibenzo[a,d]cyclohepten-5-ylidene)-1-piperidinyl]-N-(2-phenylethyl)-hexanaminedihydrochloride

Prepared by the method of Example 3, using 6-oxo-6-[(2-phenylethyl)amino]hexanoic. Mp 255-256°C (methanol, 2-propanol, ethyl acetate).

Example 30

3-Dibenz[b,e]oxepin-11(6H)-ylidene-N-methyl-N-[6-[4-(1,1-dimethylethyl)phenyl]-6-oxohexyl]-1-propanamine hydrochloride

To a stirred solution of 3-dibenz[b,e]oxepin-11(6H)-ylidene-N-methyl-1-propanamine (10.0 g, 0.033 mol) in DMF (80 ml) were added 6-bromo-1-[4-(1,1-dimethylethyl)phenyl]-1-hexanone (10.3 g, 0.033 mo[) and potassium bicarbonate (6.6 g, 0.066 mol). The mixture was heated to 75-80°C under nitrogen for 28 hours. The reaction was cooled to ambient temperature, diluted with water (300 ml) and extracted with ethyl

acetate (2 x 150 ml). The combined ethyl acetate extracts were washed with water (3 x 150 ml) and saturated NaCl (100 ml) and dried over magnesium sulfate. Removal of solvent gave an oil of 17.1 g. This oil was dissolved in 2-propanol (100 ml) and acidified with HCl gas. The solution was diluted to 400 ml with anhydrous ether and cooled. The solid was collected by filtration, recrystallized twice from ethyl acetate-methanol (15:1), and vacuum dried at 70° C for 92 hours to provide 5.69 g of 3-dibenz[b,e]oxepin-11(6H)-ylidene-N-methyl-N-[6-[4-(1,1-dimethylethyl)phenyl]-6-oxohexyl]-1-propanamine hydrochloride, mp 159-161° C.

Example 31

3-Dibenz[b,e]oxepin-11(6H)-ylidene-N-methyl-N-[4-(1,1-dimethylethyl)phenyl]-4-oxobutyl]-1-propanamine hydrochloride

To a solution of 3-dibenz[b,e]oxepin-11(6H)-ylidene-N-methyl-1-propanamine (12.2 g, 0.046 mol) in toluene (100 ml) were added potassium bicarbonate (8.0 g, 0.08 mol), 1-[4-(1,1-dimethylethyl)phenyl-4-chloro-1-butanone (12.1 g, 0.051 mol), and potassium iodide (0,6 g, 0.0036 mol).

The mixture was heated to 95-100° C for 80 hours under a nitrogen atmosphere. The solution was cooled to ambient temperature, diluted with water (200 ml) and extracted with ethyl acetate (2 x 200 ml). The combined ethyl acetate extracts were washed with saturated NaCl and dried over $MgSO_4$. Removal of solvent gave an oil of 21.7 g. This oil was dissolved in 2-propanol (100 ml) and acidified with HCl gas. Ether (150 ml) was added and the solid which formed was collected by filtration to give an off-white solid of 13.9 g.

The above solid was treated with 5% sodium hydroxide (100 ml) and extracted with chloroform (2 x 100 ml). The combined chloroform extracts were dried and evaporated to give an oil of 12.8 g. The above oil was purified by chromatography on a Waters Prep 500 HPLC on silica gel, eluting with 3% ammoniated methanol-chloroform. The fractions containing the desired products were combined and the solvents removed to give 10.7 g of an oil.

The above oil was dissolved in 2-propano[ (100 ml) and acidified with HCl gas. Ether (250 ml) was added and the solid was collected by filtration to give 11.3 g of a white solid, mp 198-203° C. A 6.5 g sample of the above solid was recrystallized from 2-propanol:methanol (3:2) and vacuum dried at 80° C for 48 hours to provide 5.0 g of 3-dibenz[b,e]oxepin-11(6H)-ylidene-N-methyl-N-[4-[4-(1,1-dimethylethyl)-phenyl]-4-oxobutyl]-1-propanamine hydrochloride; mp 209-212° C.

Example 32

3-Dibenz[b,e]oxepin-11(6H)-ylidene-N-methyl-N-[4-hydroxy-4-[4-(1,1-dimethylethyl)phenyl]butyl]-1-propanamine-4-methylbenzenesulfonic acid (1:1) salt

To a stirred solution of 3-dibenz[b,e]oxepin-11-(6H)ylidene-N-methyl-N-[4-[4-(1,1-dimethylethyl)phenyl]-4-oxobutyl]-1-propanamine hydrochloride (6.1 g, 0.013 mol) in methanol (100 ml] was added enough 15% sodium hydroxide to give a basic solution. The solution was cooled in an ice-water bath, under a nitrogen atmosphere, and sodium borohydride (0.96 g, 0.025 mol) was added in portions. The reaction slowly warmed to ambient temperature and was stirred at that temperature for 3 hours. Acetone (10 ml) was added and the solvents were removed under vacuum. The residue was dissolved in water (100 ml) and extracted with dichloromethane (3 x 100 ml). The combined dichloromethane extracts were washed with saturated NaCl (100 ml), dried and the solvent evaporated to give 6.3 g of an oil. The above oil was purified by chromatography on a Waters Prep 500 HPLC on silica gel eluting with 3% ammoniated methanol-chloroform. The fractions containing the desired product were combined and the solvents evaporated to give 5.6 g of an oil. This oil was dissolved in ethyl acetate (100 ml), decolorized with carbon, and the solvent removed to give 4.9 g of a colorless oil.

The above oil was dissolved in methanol (100 ml) and 4-methylbenzenesulfonic acid monohydrate (2.0 g, 0.01 mol) was added. The solution was stirred at ambient temperature for a few minutes and the solvent was removed. The residue was dissolved in ethyl acetate (30 ml) and ether (15 ml). The solid which formed was collected by filtration and vacuum dried at 30° C for 72 hours to provide 3.9 g of 3-dibenz[b,e]oxepin-11(6H)-ylidene-N-methyl-N-[4-hydroxy-4-[4-(11-dimethylethyl)phenyl]butyl]-1-propanamine 4-methylbenzenesulfonic acid (1:1) salt, mp 140-144 °C.

Example 33

3-Dibenz[b,e]oxepin-11(6H)-ylidene-N-methyl-N-[6-[4-[1,1-dimethylethyl)phenyl]hexyl]-1-propanamine hydrochloride

To a stirred solution of 6-[4-(1,1-dimethylethyl)phenyl]hexanoic acid (10.5 g, 0.0432 mol) in tetrahydrofuran (150 ml) were added N-hydroxysuccinimide (4.86 g, 0.0423 mo[) and dicyclohexylcarbodiimide (8.72 g, 0.0423 mol). The reaction was stirred at ambient temperature under nitrogen for 1.5 hours. The precipitated solid was removed by filtration. To a stirred solution of the filtrate, at ambient temperature under nitrogen, was added a solution of 3-dibenz[b,e]oxepin-11(6H)-ylidene-N-methyl-1-propanamine (11.2 g, 0.0423 mol) in tetrahydrofuran (120 ml) and the reaction was stirred at ambient temperature for 22 hours. The mixture was filtered, the solvent evaporated, and the residue dissolved in ethyl acetate (250 ml). The ethyl acetate solution was washed with water (1 x 200 ml), 1N HCl (200 ml), 2N sodium carbonate (200 ml), and saturated NaCl (200 ml) and dried over magnesium sulfate. Removal of solvent gave 26.2 g of an oil. The above oil was purified by silica gel chromatography on a Waters Prep 500 HPLC, eluting with chloroform. The fractions containing the desired product were combined and the solvents evaporated to give 15.4 g of 3-dibenz[b,e]oxepin-11(6H)-ylidene-N-methyl-N-[6-[4-(1,1-dimethylethyl)phenyl]-1-oxohexyl]-11propanamine as an oil.

To a stirred solution of the above oil (15.2 g, 0.0307 mol) in anhydrous ether (700 ml) at 0 °C under nitrogen was added lithium aluminium hydride (6.90 g, 0.182 mol) and the mixture was stirred for 45 min. Water (7 ml), 15% NaOH (7 ml) and water (21 ml) were carefully added. The reaction mixture was warmed to ambient temperature and the precipitated solids were removed by filtration. The filtrate was concentrated under vacuum to give 12.38 g of an oil. This oil was dissolved in 2-propano] (30 ml) and ether (170 ml) and acidified with HCl gas. The solid was collected by filtration, recrystallized from ethyl acetate (150 ml) and methanol (20 ml), and vacuum dried at 70 °C for 48 hours to provide 6.93 g of 3-dibenz[b,e]oxepin-11(6H)-ylidene-N-methyl-N-[6-[4-(1,1-dimethylethyl)phenyl]hexyl]-1-propanamine hydrochloride, mp 161-163 °C.

Example 34

3-Dibenz[b,e]oxepin-11(6H)-ylidene-N-methyl-N-[4-[4-(1,1-dimethylethyl)phenyl]butyl]-1-propanamine hydrochloride

Prepared by the method of Example 30, using 1-bromo-4-[4-(1,1- dimethylethyl)phenyl]butane. Mp 184-186 °C.

Or, prepared by the method of Example 33, using 4-[4-(1,1- dimethylethyl)phenyl]butanoic acid. Mp 184-186 °C.

Example 35

3-Dibenz[b,e]oxepin-11(6H)-ylidene-N-methyl-N-[5-[4-(1,1-dimethylethyl)phenyl]-5-oxopentyl]-1-propanamine hydrochloride

Prepared by the method of Example 30, using 5-chloro-1-[4-(1,1- dimethylethyl)phenyl]-1-pentanone. Mp 42-145 °C.

Example 36

3-Dibenz[b,e]oxepin-11(6H)-ylidene-N-methyl-N-[5-[4-(1,1-dimethylethyl)phenyl]pentyl]-1-propanamine hydrochloride

Prepared by the method of Example 33, using 5-[4-(1,1-dimethylethyl)phenyl]pentanoic acid. Mp 150-151 °C.

Example 37

3-Dibenz[b,e]oxepin-11(6H)-ylidene-N-methyl-N-[5-hydroxy-5-[4-(1,1-dimethylethyl)phenyl]pentyl]-1-propanamine

Prepared by the method of Example 32, using 3-dibenz[b,e]oxepin-11(6H)-ylidene-N-methyl-N-[5-[4-(1,1-dimethylethyl)phenyl]-5-oxopentyl]-1-propanamine.
NMR δ, 1.2-2.6 (m, 13H), 1.33 (s, 9H), 2.13, 2.25 (two s (5:1 ratio), 3H), 4.5-5.8 (broad S, 2H), 4.63 (t, J = 7Hz, 1H), 6.06, 5.72 (two t (5:1 ratio), J = 7Hz, IH), 6.77 (d, J = 8Hz, IH), 6.88 (t, J = 8Hz, IH), 7.0-7.5 (m, IOH).

Example 38

3-Dibenz[b,e]oxepin-11(6H)-ylidene-N-methyl-N-[6-hydroxy-6-[4-(1,1-dimethylethyl)phenyl]hexyl]-1-propanamine

Prepared by the method of Example 32, using 3-dibenz[b,e]oxepin-11(6H)-ylidene-N-methyl-N-[6-[4-(1,1-dimethylethyl]phenyl]-6-oxohexyl]-1-propanamine hydrochloride. Mp 73-75 °C.

Example 39

E-3-Dibenz[b,e]oxepin-11(6H)-ylidene-N-methyl-N-[4-[4-(1,1-dimethylethyl)phenyl]-4-oxobutyl]-1-propanamine hydrochloride

Prepared by the method of Example 31, using E-3-dibenz[b,e]oxepin-11(6H)-ylidene-N-methyl-1-propanamine. Mp 217-218 °C.

## Claims

1. A compound of formula I:

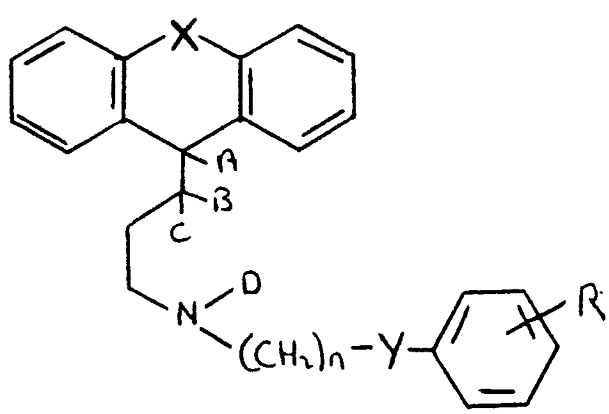

I

wherein:
X represents $-CH_2O-$, $-CH_2CH_2-$ or $-CH=CH-$;
either A represents -OH and B represents hydrogen, or A and B taken together form a second bond between the carbons to which they are attached;
either C represents hydrogen and D represents C1 to 6 alkyl, or C and D form a saturated two carbon chain.
Y represents $-CH_2-$, $-C(=O)-$, $-CH(OH)-$, -S-, -NH-, -o-, or $-NHCH_2CH_2-$;
R represents C1 to 6 alkyl, $-C(CH_3)_2CH_2OH$, $-C(CH_3)_2CO_2H$, $-C(CH_3)_2COOR'$; and may be at the 2, 3 or 4 position of the benzene ring relative to the rest of the molecule,
R′ represents C1 to 6 alkyl
and n represents an integer between 3 and 6,
and pharmaceutically acceptable acid addition salts thereof.

2. A compound according to Claim 1 where R is tert butyl.

3. A compound according to Claim 1 where A and B together form a second bond between the carbons to which they are attached.

4. A compound according to Claim 1 wherein X represents $-CH_2O-$, $-CH=CH-$ or $-CH_2CH_2-$; C and D together form a $CH_2CH_2$ chain; A represents hydroxy when B represents hydrogen, or A and B taken together form a second bond between the carbons to which they are attached; n is an integer between 3 and 6; Y represents $-C(=O)-$, $-CH_2-$, $-CH(OH)-$, -S-, -NH-, -O- or $-NHCH_2CH_2-$; R represents tert butyl, $-C(CH_3)_2COOR'$ where R′ represents C1 to 6 alkyl or $-C(CH_3)_2CH_2OH$.

5. A process for the preparation of compounds of formula I which comprises:
a) reacting an amine of formula II:

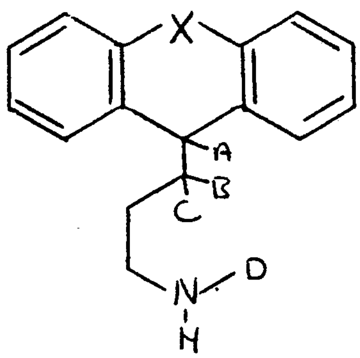

II

with an alkylation agent of formula III:

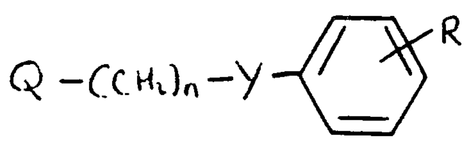

III

wherein Q is chlorine or bromine;
b) reducing an amide of formula IV:

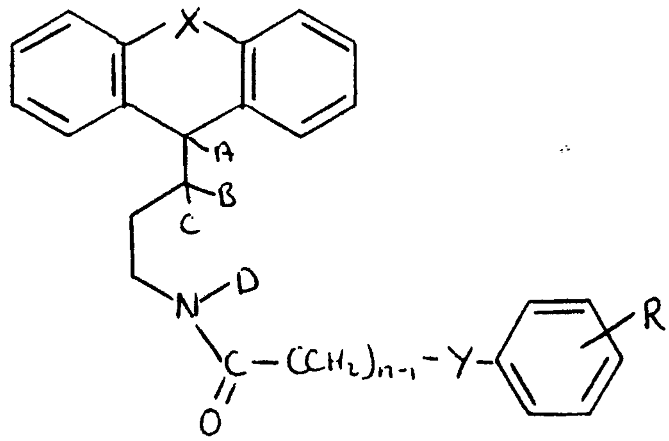

IV

c) producing a compound of formula I in which Y is -CH(OH)-, by reduction of the corresponding compound of formula I in which Y is -C( = O)-;

d) producing a compound if formula I in which R′ is H, by hydrolysis of the corresponding compound of formula I in which R′ is C1 to 6 alkyl;

e) producing a compound of formula I in which R′ is C1 to 6 alkyl by esterification of the corresponding compound of formula I in which R′ is H;

f) producing a compound of formula I in which R is $-C(CH_3)_2CH_2OH-$ by reduction of the corresponding compound of formula I in which R is $-(CH_3)_2COOR'$,

and, where desired or necessary, converting the compound of formula I into a pharmaceutically acceptable acid addition salt.

6. A compound according to Claim 1 wherein X represents $-CH_2O-$; A and B together form a second bond between the carbons to which they are attached; C represents hydrogen and D represents methyl; R represents tert butyl; n represents an integer between from 3 to 5 and Y represents $-CH_2-$, -CH(OH)- or -C-( = O)-.

7. The use of a compound of formula I as defined in any one of claims 1 to 6 for the manufacture of a medicament for the treatment of a human or animal patient suffering from a disease or condition mediated by the response of $H_1$ receptors to histamine.

8. A pharmaceutical composition comprising a compound of formula I, or a pharmaceutically acceptable salt thereof, in admixture with a pharmaceutically acceptable adjuvant, diluent or carrier.

9. A compound according to Claim 1 where the compound is 4-(10,11-Dihydro-5H-dibenzo[a,d]-cyclohepten-5-ylidene)-1-[4-[4-(1,1-dimethylethyl)phenyl]-4-hydroxybutyl]- piperidine.

10. A compound according to Claim 1 where the compound is: 4-[4-(10,11-Dihydro-5H-dibenzo[a,d]-cyclohepten-5- ylidene)- 1-piperidinyl]-1-[4-(1,1-dimethylethyl)phenyl]-1-butanone;
4-(10,11-Dihydro-5H-dibenzo[a,d]cyclohepten-5-ylidene)-1-[4-[4-(1,1-dimethylethyl)phenyl]butyl]piperidine;
4-Dibenz[b,e]oxepin-11(6H)-ylidene-1-[4-[4-(1,1-dimethylethyl)phenyl]butyl]piperidine;
4-[4-(10,11-Dihydro-5-hydroxy-5H-dibenzo[a,d]cyclohepten-5-yl)-1-piperidinyl]-1-[4-(1,1-dimethylethyl)-phenyl]-1-butanone;
4-(4-Dibenz[b,e]oxepin-11(6H)-ylidene-1-piperidinyl)-1-[4-(1,1-dimethylethyl)phenyl]-1-butanone;
4-Dibenz[b,e]oxepin-11(6H)-ylidene-1-[4-[4-(1,1-dimethylethyl)phenyl]-4-hydroxybutyl]piperidine;
4-[4-(6,1]-Dihydro-[1-hydroxydibenz[b,e]oxepin-11-yl)-1-piperidinyl]-1-[4-(1,1-dimethylethyl)phenyl]butanone;
11-[1-[4-[4-(1,1-Dimethylethyl)phenyl]-4-hydroxybutyl]-4-piperidinyl]-6,11-dihydrodibenz[b,e]oxepin-11-ol;
11-[1-[4-[4-(1,1-Dimethylethyl)phenyl]butyl]-4-piperidinyl]-6,11-dihydrodibenz[b,e]oxepin-11-ol;
10,11-Dihydro-5-[1-[4-[4-(1,1-dimethylethyl)phenyl]-4-hydroxybutyl]-4-piperidinyl]-5H-dibenzo[a,d]-cyclohepten-5-ol;
10,11-Dihydro-5-[1-[4-[4-(1,1-dimethylethyl)phenyl]butyl]-4-piperidinyl]-5H-dibenzo[a,d]cyclohepten-5-ol;
4-[4-(5H-Dibenz[a,d]cyclohepten-5-ylidene)-1-piperidinyl]-1-[4-(1,1-dimethylethyl)phenyl]-1-butanone;
4-(5H-Dibenzo[a,d]cyclohepten-5-ylidene)-1-[4-[4-(1,1-dimethylethyl)phenyl]-4-hydroxybutyl]piperidine;
5-[4-(10,11-Dihydro-5H-dibenzo[a,d]cyclohepten-5-ylidene)-1-piperidinyl]-1-[4-(1,1-dimethylethyl)phenyl]-1-pentanone;
6-[4-(10,11-Dihydro-5H-dibenzo[a,d]cyc]ohepten-5-ylidene)-1-piperidinyl]-1-[4-(1,1-dimethylethyl)phenyl]-1-hexanone;
4-(10,11-Dihydro-5H-dibenzo[a,d]cyclohepten-5-ylidene)-1-[5-[4-(1,1-dimethylethyl)phenyl]-5-hydroxypentyl] piperidine

4-(10,11-Dihydro-5H-dibenzo[a,d]cyclohepten-5-ylidene)-1-[6-[4-(1,1-dimethylethyl)phenyl]-6-hydroxyhexyl]-piperidine
4-(10,11-Dihydro-5H-dibenzo[a,d]cyclohepten-5-ylidene)-1-[5-[4-(1,1-dimethylethyl)phenyl]pentyl]piperidine;
4-(10,11-Dihydro-5H-dibenzo[a,d]cyclohepten-5-ylidene)-1-[6-[4-(1,1-dimethylethyl)phenyl]hexyl]piperidine;
4-(10,11-Dihydro-5H-dibenzo[a,d]cyclohepten-5-ylidene)-1-[3-[4-(1,1-dimethylethyl)phenoxy]propyl]-piperidine;
4-(10,11-Dihydro-5H-dibenzo[a,d]cyclohepten-5-ylidene)-1-[3-[[4-(1,1-dimethylethyl)phenyl]thio]propyl]-piperidine;
3-[4-(10,11-Dihydro-5H-dibenzo[a,d]cyclohepten-5-ylidene)-1-piperidinyl]-N-[4-(1,1-dimethylethyl)phenyl] propanamine;
4-[4-[4-(10,11-Dihydro-5H-dibenzo[a,d]cyclohepten-5- ylidene)-1-piperidinyl]-1-oxobutyl]-α,α-dimethylbenzene acetic acid;
4-[4-[4-(10,11-Dihydro-5H-dibenzo[a,d]cyclohepten-5-ylidene)-1-piperidinyl]-1-hydroxybutyl]-α,α-dimethylbenzene acetic acid;
4-[4-[4-(10,11-Dihydro-5H-dibenzo[a,d]cyclohepten-5-ylidene)-1-piperidinyl]-1-hydroxybutyl]-α,α-dimethylbenzene acetic acid;
4-[4-[4-(10,11-Dihydro-5H-dibenzo[a,d]cyclohepten-5-ylidene)-1-piperidinyl]-1-oxobutyl]-α,α-dimethylbenzene acetic acid;
4-(10,11-Dihydro-5H-dibenzo[a,d]cyclohepten-5-ylidene)-1-[4-[4-(1,1-dimethyl-2-hydroxyethyl)phenyl]-4-hydroxybutyl] piperidine;
6-[4-(10,11-Dihydro-5H-dibenzo[a,d]cyclohepten-5-ylidene)-1-piperidinyl]-N-(2-phenylethyl)hexanamine;
3-Dibenz[b,e]oxepin-11(6H)-ylidene-N-methyl-N-[6-[4-(1,1-dimethylethyl)phenyl]-6-oxohexyl]-1-propanamine;
3-Dibenz[b,e]oxepin-11(6H)-ylidene-N-methyl-N-[4-(1,1-dimethylethyl)phenyl]-4-oxobutyl]-1-propanamine;
3-Dibenz[b,e]oxepin-11(6H)-ylidene-N-methyl-N-[4-hydroxy-4-[4-(1,1-dimethylethyl)phenyl]butyl]-1-propanamine-4-methylbenzenesulfonic acid;
3-Dibenz[b.e]oxepin-11(6H)-ylidene-N-methyl-N-[6-[4-[1,1-dimethylethyl)phenyl]hexyl]-1-propanamine;
3-Dibenz[b,e]oxepin-11(6H)-ylidene-N-methyl-N-[4-[4-(1,1-dimethylethyl)phenyl]butyl]-1-propanamine;
3-Dibenz[b,e]oxepin-11(6H)-ylidene-N-methyl-N-[5-[4-(1,1-dimethylethyl)phenyl]-5-oxopentyl]-1-propanamine;
3-Dibenz[b,e]oxepin-11(6H)-ylidene-N-methyl-N-[5-[4-(1,1-dimethylethyl)phenyl]pentyl]-1-propanamine;
3-Dibenz[b,e]oxepin-11(6H)-ylidene-N-methyl-N-[5-hydroxy-5-[4-(1,1-dimethylethyl)phenyl]pentyl]-1-propanamine
3-Dibenz[b,e]oxepin-11(6H)-ylidene-N-methyl-N-[6-hydroxy-6-[4-(1,1-dimethylethyl)phenyl]hexyl]-1-propanamine;
E-3-Dibenz[b,e]oxepin-11(6H)-ylidene-N-methyl-N-[4-[4-(1,1-dimethylethyl)phenyl]-4-oxobutyl]-1-propanamine.

Claims for the following Contracting States: ES and GR

1. A process for the preparation of compounds of formula I:

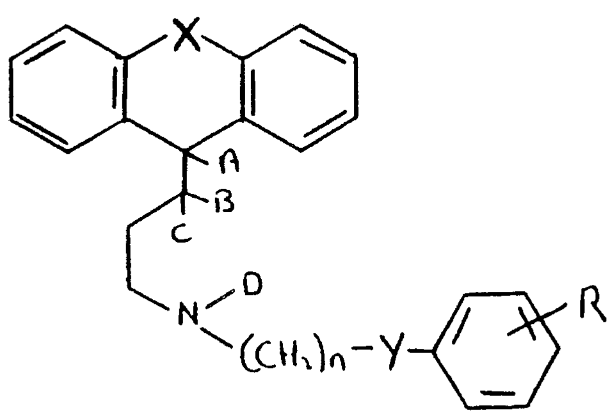

I

wherein:
X represents $-CH_2O-$, $-CH_2CH_2-$ or $-CH=CH-$;

either A represents -OH and B represents hydrogen, or A and B taken together form a second bond between the carbons to which they are attached;
either C represents hydrogen and D represents C1 to 6 alkyl, or C and D form a saturated two carbon chain.
Y represents $-CH_2-$, -C(=O)-, -CH(OH)-, -S-, -NH-, -0-, or $-NHCH_2CH_2-$;
R represents C1 to 6 alkyl, $-C(CH_3)_2CH_2OH$, $-C(CH_3)_2CO_2H$, $-C(CH_3)_2COOR'$; and may be at the 2, 3 or 4 position of the benzene ring relative to the rest of the molecule,
R′ represents C1 to 6 alkyl
and n represents an integer between 3 and 6,
which comprises:
a) reacting an amine of formula II:

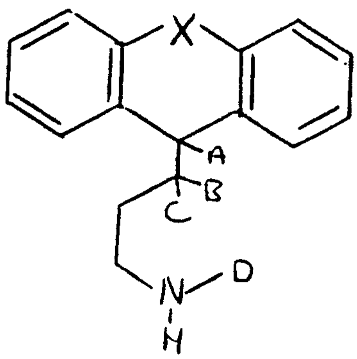

II

with an alkylation agent of formula III:

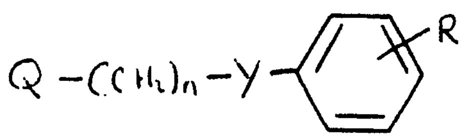

III

wherein Q is chlorine or bromine;
b) reducing an amide of formula IV:

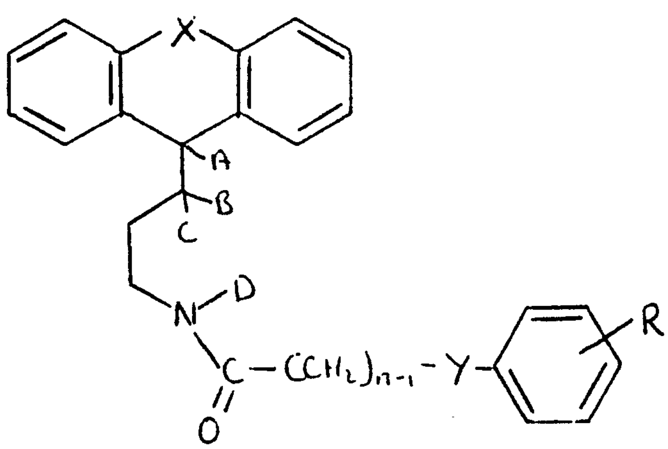

IV

c) producing a compound of formula I in which Y is -CH(OH)-, by reduction of the corresponding compound of formula I in which Y is -C(=O)-;
d) producing a compound of formula I in which R′ is H, by hydrolysis of the corresponding compound of formula I in which R′ is C1 to 6 alkyl;
e) producing a compound of formula I in which R′ is C1 to 6 alkyl by esterification of the corresponding compound of formula I in which R′ is H;
f) producing a compound of formula I in which R is $-C(CH_3)_2CH_2OH-$ by reduction of the corresponding

compound of formula I in which R is $-(CH_3)_2COOR'$,
and, where desired or necessary, converting the compound of formula I into a pharmaceutically acceptable acid addition salt.

2. A process according to Claim 1 wherein R represents C1 to 6 alkyl.

3. A process according to Claim 1 or 2 wherein C and D form a $-CH_2CH_2-$ chain.

4. A process according to any one of the preceding Claims wherein X represents $-CH_2CH_2-$.

5. A process according to any one of the preceding Claims wherein Y represents -CH(OH)-.

6. A process according to any of the preceding claims wherein A and B together form a second bond between the carbons to which they are attached.

7. A process according to Claim 1 wherein X represents $-CH_2O-$, -CH = CH- or $-CH_2CH_2-$; C and D together form a $CH_2CH_2$ chain; A represents hydroxy when B represents hydrogen, or A and B taken together form a second bond between the carbons to which they are attached; n is an integer between 3 and 6; Y represents -C( = O)-, $-CH_2-$, -CH(OH)-, -S-, -NH-, -O- or $-NHCH_2CH_2-$; R represents tert butyl, $-C(CH_3)_2COOR'$ where R′ represents C1 to 6 alkyl or $-C(CH_3)_2CH_2OH$.

8. A process according to Claim 1 where R is tert butyl.

9. A process according to Claim 1 where the compound is 4-(10,11-Dihydro-5H-dibenzo[a,d]-cyclohepten-5-ylidene)-1-[4-[4-(1,1-dimethylethyl)phenyl]-4-hydroxybutyl]- piperidine.

10. A process according to Claim 1 where the compound is:
4-[4-(10,11-Dihydro-5H-dibenzo[a,d]cyclohepten-5-ylidene)-1-piperidinyl]-1-[4-(1,1-dimethylethyl)phenyl]-1-butanone;
4-(10,11-Dihydro-5H-dibenzo[a,d]cyclohepten-5-ylidene)-1-[4-[4-(1,1-dimethylethyl)phenyl]butyl]piperidine;
4-Dibenz[b,e]oxepin-11(6H)-ylidene-1-[4-[4-(1,1-dimethylethyl)phenyl]butyl]piperidine;
4-[4-(10,11-Dihydro-5-hydroxy-5H-dibenzo[a,d]cyclohepten-5-yl)-1-piperidinyl]-1-[4-(1,1-dimethylethyl)-phenyl]-1-butanone;
4-(4-Dibenz[b,e]oxepin-11(6H)-ylidene-1-piperidinyl)-1-[4-(1,1-dimethylethyl)phenyl]-1-butanone;
4-Dibenz[b,e]oxepin-11(6H)-ylidene-1-[4-[4-(1,1-dimethylethyl)phenyl]-4-hydroxybutyl]piperidine;
4-[4-(6,1)-Dihydro-[1-hydroxydibenz[b,e]oxepin-11-yl)-1-piperidinyl]-1-[4-(1,1-dimethylethyl)phenyl]butanone;
11-[1-[4-[4-(1,1-Dimethylethyl)phenyl]-4-hydroxybutyl]-4-piperidinyl]-6,11-dihydrodibenz[b,e]oxepin-11-ol;
11-[1-[4-[4-(1,1-Dimethylethyl)phenyl]butyl]-4-piperidinyl]-6,11-dihydrodibenz[b,e]oxepin-11-ol;
10,11-Dihydro-5-[1-[4-[4-(1,1-dimethylethyl)phenyl]-4-hydroxybutyl]-4-piperidinyl]-5H-dibenzo[a,d]-cyclohepten-5-ol;
10,11-Dihydro-5-[1-[4-[4-(1,1-dimethylethyl)phenyl]butyl]-4-piperidinyl]-5H-dibenzo[a,d]cyclohepten-5-ol;
4-[4-(5H-Dibenz[a,d]cyclohepten-5-ylidene)-1-piperidinyl]-1-[4-(1,1-dimethylethyl)phenyl]-1-butanone;
4-(5H-Dibenzo[a,d]cyclohepten-5-ylidene)-1-[4-[4-(1,1-dimethylethyl)phenyl]-4-hydroxybutyl]piperidine;
5-[4-(10,11-Dihydro-5H-dibenzo[a,d]cyclohepten-5-ylidene)-1-piperidinyl]-1-[4-(1,1-dimethylethyl)phenyl]-1-pentanone;
6-[4-(10,11-Dihydro-5H-dibenzo[a,d]cyc]ohepten-5-ylidene)-1-piperidinyl]-1-[4-(1,1-dimethylethyl)phenyl]-1-hexanone;
4-(10,11-Dihydro-5H-dibenzo[a,d]cyclohepten-5-ylidene)-1-[5-[4-(1,1-dimethylethyl)phenyl]-5-hydroxypentyl] piperidine
4-(10,11-Dihydro-5H-dibenzo[a,d]cyclohepten-5-ylidene)-1-[6-[4-(1,1-dimethylethyl)phenyl]-6-hydroxyhexyl]-piperidine
4-(10,11-Dihydro-5H-dibenzo[a,d]cyclohepten-5-ylidene)-1-[5-[4-(1,1-dimethylethyl)phenyl]pentyl]piperidine;
4-(10,11-Dihydro-5H-dibenzo[a,d]cyclohepten-5-ylidene)-1-[6-[4-(1,1-dimethylethyl)phenyl]hexyl]piperidine;
4-(10,11-Dihydro-5H-dibenzo[a,d]cyclohepten-5-ylidene)-1-[3-[4-(1,1-dimethylethyl)phenoxy]propyl]-piperidine;
4-(10,11-Dihydro-5H-dibenzo[a,d]cyclohepten-5-ylidene)-1-[3-[[4-(1,1-dimethylethyl)phenyl]thio]propyl]-piperidine;
3-[4-(10,11-Dihydro-5H-dibenzo[a,d]cyclohepten-5-ylidene)-1-piperidinyl]-N-[4-(1,1-dimethylethyl)phenyl]-propanamine;
4-[4-[4-(10,11-Dihydro-5H-dibenzo[a,d]cyclohepten-5-ylidene)-1-piperidinyl]-1-oxobutyl]-α,α-dimethylbenzene acetic acid;
4-[4-[4-(10,11-Dihydro-5H-dibenzo[a,d]cyclohepten-5-ylidene)-1-piperidinyl]-1-hydroxybutyl]-α,α-dimethylbenzene acetic acid;
4-[4-[4-(10,11-Dihydro-5H-dibenzo[a,d]cyclohepten-5-ylidene)-1-piperidinyl]-1-hydroxybutyl]-α,α-dimethylbenzene acetic acid;
4-[4-[4-(10,11-Dihydro-5H-dibenzo[a,d]cyclohepten-5-ylidene)-1-piperidinyl]-1-oxobutyl]-α,α-dimethyl-benzene acetic acid;

4-(10,11-Dihydro-5H-dibenzo[a,d]cyclohepten-5-ylidene)-1-[4-[4-(1,1-dimethyl-2-hydroxyethyl)phenyl]-4-hydroxybutyl]piperidine;
6-[4-(10,11-Dihydro-5H-dibenzo[a,d]cyclohepten-5-ylidene)-1-piperidinyl]-N-(2-phenylethyl)hexanamine;
3-Dibenz[b,e]oxepin-11(6H)-ylidene-N-methyl-N-[6-[4-(1,1-dimethylethyl)phenyl]-6-oxohexyl]-1-propanamine;
3-Dibenz[b,e]oxepin-11(6H)-ylidene-N-methyl-N-[4-(1,1-dimethylethyl)phenyl]-4-oxobutyl]-1-propanamine;
3-Dibenz[b,e]oxepin-11(6H)-ylidene-N-methyl-N-[4-hydroxy-4-[4-(1,1-dimethylethyl)phenyl]butyl]-1-propanamine-4-methylbenzenesulfonic acid;
3-Dibenz[b,e]oxepin-11(6H)-ylidene-N-methyl-N-[6-[4-[1,1-dimethylethyl)phenyl]hexyl]-1-propanamine;
3-Dibenz[b,e]oxepin-11(6H)-ylidene-N-methyl-N-[4-[4-(1,1-dimethylethyl)phenyl]butyl]-1-propanamine;
3-Dibenz[b,e]oxepin-11(6H)-ylidene-N-methyl-N-[5-[4-(1,1-dimethylethyl)phenyl]-5-oxopentyl]-1-propanamine;
3-Dibenz[b,e]oxepin-11(6H)-ylidene-N-methyl-N-[5-[4-(1,1-dimethylethyl)phenyl]pentyl]-1-propanamine;
3-Dibenz[b,e]oxepin-11(6H)-ylidene-N-methyl-N-[5-hydroxy-5-[4-(1,1-dimethylethyl)phenyl]pentyl]-1-propanamine
3-Dibenz[b,e]oxepin-11(6H)-ylidene-N-methyl-N-[6-hydroxy-6 [4-(1,1-dimethylethyl)phenyl]hexyl]-1-propanamine;
E-3-Dibenz[b,e]oxepin-11(6H)-ylidene-N-methyl-N-[4-[4-(1,1-dimethylethyl)phenyl]-4-oxobutyl]-1-propanamine;